## Europäisches Patentamt

(19) **European Patent Office**

**Office européen des brevets**

(11) Numéro de publication: **0 036 350
B1**

(12) **FASCICULE DE BREVET EUROPEEN**

(45) Date de publication du fascicule du brevet:
30.11.83

(51) Int. Cl.³: **A 61 K 9/54, A 61 K 9/22**

(21) Numéro de dépôt: **81400325.7**

(22) Date de dépôt: **03.03.81**

(54) Composition pharmaceutique à activité régulatrice sur le tonus vasculaire.

(30) Priorité: **03.03.80 FR 8004728**

(43) Date de publication de la demande:
**23.09.81 Bulletin 81/38**

(45) Mention de la délivrance du brevet:
**30.11.83 Bulletin 83/48**

(84) Etats contractants désignés:
**AT BE CH DE FR GB IT LI LU NL SE**

(56) Documents cités:
**DE - A - 1 467 976
FR - A - 2 453 640
FR - A - 2 453 642
GB - A - 1 443 923
GB - A - 2 025 227**

**PH.LIST "ARZNEIFORMLEHRE" (1976, pp. 457-462
Kirk & OTHMER "Enzyclopedia of Chemical
Technology" (1978), Vol. 1, p 911
E.J. ARIENS "DRUG DESIGN" (1973), Vol. 4, p. 48-58**

(73) Titulaire: **PIERRE FABRE S.A., 125, rue de la
Faisanderie, F-75116 Paris (FR)**

(72) Inventeur: **Darbeau Daniel, 125, rue de la Faisanderie,
F-75016 Paris (FR)**

(74) Mandataire: **Martin, Jean-Jacques et al, Cabinet
REGIMBEAU 26, Avenue Kléber, F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

## Composition pharmaceutique a activite regulatrice sur le tonus vasculaire

La présente invention concerne une composition pharmaceutique dotée d'une activité régulatrice sur le tonus vasculaire, dont le principe actif est constitué par un alcaloide de l'ergot de seigle ou par l'un de ses sels.

Dans un tel type de médicament administrable par voie orale, on a déjà recherché diverses solutions pour mettre au point une présentation du médicament assurant une libération contrôle du principe actif. Les documents de technique antérieure suivants peuvent servir d'illustration de l'état de la technique : GB-A-2 025 227 — P. H. List, »Arzneiformenlehre«, 1976, Wissenschaftliche Verlagsgesellschaft, Stuttgart, pages 457 à 462 — Kirk-Othmer »Encyclopedia of Chemical Technology«, 3ème édition, volume 1, 1978, Wiley-Interscience Publication, New York, page 911 — E. J. Ariens, »Drug Design«, volume 4, 1973, Academic Press, New York, pages 48 à 58.

Le problème que vise à résoudre la présente invention est d'assurer l'obtention d'une dose de principe actif dans l'organisme qui reste la plus constante possible, tout en diminuant la fréquence des administrations. En d'autres termes, la composition selon la présente invention a pour but d'assurer la meilleure utilisation possible d'une certaine quantité du principe actif administré, c'est-à-dire d'obtenir un effet pharmacologique supérieur à la fois en efficacité et en durée aux effets obtenus avec les compositions d'alcaloides d'ergot de seigle de la technique antérieure.

Conformément à la présente invention, la composition pharmaceutique se présente sous la forme de doses unitaires de 3 à 10 mg d'un principe actif qui est un alcaloide de l'ergot de seigle ou un de ses sels et ayant un effet étalé dans le temps, caractérisée en ce que ledit principe actif se présente sous forme de micro-granulés, les uns contenant 10 à 20% en poids de la dose totale du principe actif étant à libération instantanée, les autres, contenant de 80 à 90% en poids du principe actif, étant protégés par plusieurs enrobages gastro-solubles et gastro-résistants à délitement programmé en fonction du pH et ayant un temps de dissolution totale théorique in vitro compris entre 10 et 14 heures.

D'autres caractéristiques et avantages de la composition selon l'invention apparaitront à la lecture de la description détaillée faite ci-après en référence à quelques exemples de modes de réalisation particuliers donnés à simple titre d'illustration.

La composition selon la présente invention, plus particulièrement dotée d'une activité régulatrice sur le tonus vasculaire, est destinée à être administrée par voie orale. Une telle composition se présente sous la forme de doses unitaires d'administration contenant chacune environ 3 à environ 10 mg d'un principe actif constitué par un alcaloïde de l'ergot de seigle ou par l'un de ses sels. Le principe actif entrant dans la composition de l'invention peut en particulier être constitué par un alcaloïde de l'ergot de seigle à fragment acide lysergique dihydrogéné, tel que la dihydroergotamine, ou l'un de ses sels tel que par exemple le méthane sulfonate ou le bitartrate.

De manière à obtenir le meilleur effet pharmacologique possible, le principe actif de la composition selon l'invention a été réparti à raison d'environ 80 à 90% en poids sous une forme à libération programmée dont le temps de dissolution total théorique in vitro est sensiblement compris entre 10 et 14 heures, le complément se présentant sous une forme entièrement à libération instantanée.

La libération programmée du principe actif est déterminée en fonction des valeurs de pH du milieu de dissolution. Cette détermination in vitro fournit des temps de dissolution théorique du principe actif. A titre d'illustration de cette technique bien connue, on précisera par exemple que l'on peut déterminer cette libération programmée pour les valeurs habituelles de pH suivantes:

| | | |
|---|---|---|
| milieu gastrique artificiel | pH = | 1,5 |
| milieux intestinaux artificiels successifs | pH = | 4,5 |
| | pH = | 6,9 |
| | pH = | 7,2 |
| | pH = | 7,5 |

D'autre part, on condidère habituellement qu'une forme de principe actif est dite entièrement à libération instantanée lorsque le délitement de la totalité du principe actif se réalise à l'intérieur d'une période de 10 minutes.

La composition selon la présente invention peut en particulier se présenter sous la forme de doses unitaires d'environ 3 à 10 mg de principe actif, affectant notamment la forme de micro-granulés rassemblés au sein de capsules dures ou gélules, ou encore de comprimés.

De tels micro-granulés et en particulier les microgranulés à action programmée, se présentent sous la forme de sphérules multicouches comprenant chacune un coeur sur lequel est appliquée, par exemple par pulvérisation, une succession de couches de principe actif et d'excipients d'enrobage.

Ces micro-granulés sont préparés par des techniques bien connues de l'homme de l'art, dont on rappellera brièvement les principales caractéristiques de fabrications. Dans un premier temps, on prépare les coeurs neutres des micro-granulés. Ces coeurs neutres peuvent par exemple être réalisés sous la forme de minuscules grains composites de saccharose et d'amidon de mais. En pratique, il est important de leur conférer une forme la plus sphérique possible, et de préparer des micro-granulés uniformément égaux. La préparation des coeurs neutres des micro-granulés est habituellement mise en oeuvre dans des mélangeurs spéciaux à réglage thermostatique. On pulvérise ensuite sur ces coeurs sphériques neutres successivement un certain nombre de couches de principe actif et d'excipient d'enrobage. Les diverses opérations de pulvérisation s'effectuent habituellement dans des turbines qui permettent de conserver la forme sphérique des micro-granulés au cours des dépôts successifs de couches et, partant, d'obtenir des couches uniformément égales. Bien entendu, il convient de bien séparer les diverses opérations successives de dépôt, entre lesquelles on procède habituellement à une opération de séchage par circulation forcée d'air dont la température est contrôlée thermostatiquement.

Dans la pratique le principe actif, par exemple le méthane sulfonate de dihydroergotamine, est pulvérisé sur les coeurs neutres des micro-granulés, par exemple sous la forme de solutions avantageusement dotées de propriétés collantes. Le principe actif peut notamment être appliqué par pulvérisation en mélange à des excipients divers parmi lesquels on citera à titre d'exemple les solutions alcooliques de polyvinyl-pyrrolidone.

Les divers enrobages gastro-solubles et gastrorésistants sont choisis expérimentalement, pour conduire à un délitement programmé de type particulier, par exemple en fonction du pH. L'ordre, le nombre, l'épaisseur et la nature des couches d'enrobage des micro-granulés permettent de programmer expérimentalement in vitro le temps de délitement. A titre d'exemples de substances d'enrobage, on mentionnera les gommes, les dérivés de la cellulose tels que l'éthylcellulose et l'acétophtalate de cellulose, additionnés éventuellement de plastifiants convenables, les résines vinyliques ou acryliques notamment les résines commercialisées sous la dénomination »Eudragit®« par la Société allemande Rohm Pharma, les mélanges de cires, les glycérides, les stéarates de glycérol ou de sorbitol, la gélatine formolée, etc. En pratique on sait que les micro-granulés sous forme à libération programmée présentent ainsi une ou plusieurs couches d'enrobage gastro-soluble ou gastro-résistant d'épaisseur uniforme déterminée. Etant donné que les sucs gastro-Intestinaux au contact de ces couches d'enrobage des micro-granulés vont solubiliser le principe actif de façon progressive et réglée en fonction du temps et du pH, le choix des substances d'enrobage s'effectuera précisément en fonction de leurs caractéristiques de solubilisation par rapport au pH. De cette manière, il est donc possible d'obtenir un délitement progressif des micro-granulés en fonction des variations physiologiques du pH, tout au long du transit gastrointestinal.

A titre d'exemple particulier, on indiquera ci-après le calcul et les normes de libération programmée de micro-granulés de méthane sulfonate de dihydroergotamine, sous forme à libération programmée dont le temps de dissolution total théorique in vitro est de 12 heures.

Pour doser le pourcentage de méthanesulfonate de dihydroergotamine libéré en fonction du temps, on utilisera par exemple les réactifs suivants:

- milieu gastrique artificiel à pH 1,5 à 37°:
  - chlorure de sodium                                          2 g
  - acide chlorhydrique dilué à 10% (p/p), q.s.p.              pH 1,5
  - eau purifiée, q.s.p.                                        1000 ml
- milieux intestinaux artificiels
  a) milieu à pH 4,5 à 37°
     - phosphate monopotassique (R)                            6,8 g
     - solution aqueuse N d'hydroxyde de sodium, q.s.p.        pH 4,5
     - eau purifiée, q.s.p.                                     1000 ml
  b) milieu à pH 6,9 à 37°
     - Phosphate monopotassique                                3,4 g
     - phosphate disodique anhydre                             3,55 g
     - solution aqueuse N d'hydroxyde de sodium, q.s.p.        pH 6,9
     - eau purifiée, q.s.p.                                     1000 ml
  c) milieu à pH 7,2 à 37°
     - phosphate monopotassique (R)                            6,8 g
     - solution aqueuse N d'hydroxyde de sodium, q.s.p.        pH 7,2
     - eau purifiée, q.s.p.                                     1000 ml
  d) milieu à pH 7,5 à 37°
     - phosphate monopotassique (R)                            6,8 g
     - solution aqueuse N d'hydroxyde de sodium, q.s.p.        pH 7,5
     - eau purifiée, q.s.p.                                     1000 ml

L'appareillage de dosage utilisé est un dispositif extracteur rotatif, par exemple du type »Diffutest®« de la Société Eurand. Un tel appareil est contenu dans une armoire thermostatée à 37°C et sa vitesse de rotation est de 30 tours par minute. Il est muni de flacons en verre de 40 ml, formés de deux parties:

- 1 tube en verre ouvert aux deux extrémités sur lequel on ajuste à la partie inférieure,
- un fritté, lui-même recouvert d'un filtre en non tissé.

La partie supérieure du tube en verre et le fritté sont bouclıés à l'emeri.

Le produit à tester est introduit dans ces flacons, ainsi que la quantité de solution nécessaire (40 ml) pour chaque phase de l'analyse. Ensuite, chaque flacon hermétiquement clos est placé sur le porte tube rotatif, on fait tourner celui-ci à vitesse constante durant la période nécessaire pour chaque phase de l'analyse. A la fin de chaque période de rotation, le liquide contenu dans le flacon est transféré dans un récipient adéquat par filtration au moyen d'une trompe à vide. A la fin de chaque phase, les granules et la partie interne du flacon sont lavés avec 5 ml d'eau purifiée.

Technique opératoire

Dans un flacon, on introduit une prise d'essai exactement pesée voisine de 330 mg de microgranules de la composition selon l'invention. On ajoute 40 ml de milieu gastrique pH 1,5. On agite pendant 10 minutes. On filtre. On recueille le filtrat dans une fiole jaugée de 100 ml ($A_1$).

On lave le flacon contenant les microgranules avec 5 ml d'eau purifiée. On ajoute 20 ml d'alcool éthylique à 95° et 2 ml d'acide chlorhydrique (R) au filtrat et on complète à 100 ml avec de l'eau purifiée (solution $A_1$).

On introduit 40 ml de milieu pH 1,5 dans le flacon contenant les microgranules et on agite pendant 50 mn. On recueille le liquide dans une fiole de 100 ml ($A_2$) en procédant comme précédemment. Après le lavage, on ajoute 20 ml d'alcool éthylique à 95° et 2 ml d'acide chlorhydrique au filtrat et on complète à 100 ml avec de l'eau purifiée (solution $A_2$).

On introduit 40 ml de milieu à pH 4,5 dans le flacon contenant les microgranules, on agite pendant l heure et on recueille le liquide dans une fiole de 200 ml (B) comme précédemment. Après lavage des microgranules, on laisse en attente.

On introduit 40 ml de milieu à pH 6,9 dans le flacon contenant les microgranules et on agite pendant 2 heures. On recueille le liquide dans la fiole de 200 ml (B) contenant l'extrait précédent.

Après lavage, on ajoute 40 ml d'alcool éthylique à 95° et 4 ml d'acide chlorhydrique (R) à la fiole jaugée, puis on complète à 200 ml avec de l'eau purifiée (solution B).

On introduit 40 ml de milieu à pH 6,9 dans le flacon contenant les microgranules et on agite pendant l heure. On recueille le liquide dans une fiole jaugée de 200 ml (C). On lave et on laisse en attente.

On introduit 40 ml de milieu à pH 7,2 dans le flacon contenant les microgranules, on agite pendant 2 heures et on recueille le liquide dans la fiole (C) contenant l'extrait précédent. On laisse en attente.

On introduit 40 ml de milieu à pH 7,5 dans le flacon contenant les microgranules, on agite pendant l heure et on recueille le liquide dans la fiole (C). On ajoute 40 ml d'alcool éthylique à 95° et 4 ml d'acide chlorhydrique. On complète à 200 ml avec de l'eau purifiée (solution C).

On introduit 40 ml de milieu à pH 7,5 dans le flacon contenant les microgranules, on agite pendant 4 heures et on recueille le liquide dans une fiole jaugée de 100 ml. On ajoute 20 ml d'alcool éthylique à 95° et 2 ml d'acide chlorhydrique (R). On complète à 100 ml avec de l'eau purifiée (solution D).

On filtre les solutions $A_1$, $A_2$, B, C et D.

On effectue la lecture de chaque solution au spectrophotomètre à 280 nm ± 2 et à 320 nm ± 2.

On fait le zéro de l'appareil sur la solution de dilution.

On examine le témoin dans les mêmes conditions.

L'essai est réalisé sur 6 prélèvements.

Calcul et normes de libération

On calcule la quantité et le pourcentage de méthanesulfonate de dihydroergotamine contenu dans chacune des fioles, soit respectivement: a1, a2, b, c et d pour les fioles $A_1$, $A_2$, B, C et D.

X est la quantité de dihydroergotamine méthanesulfonate contenue dans 330 mg de microgranules retardés.

Le pourcentage de produit libéré est de:

— après 10 minutes $\dfrac{a1 \times 100}{X}$

rorme: comprise entre 10% et 20% (de préffäérence de 10 à 16%)

— après 1 heure $\dfrac{(a1 + a2) \times 100}{X}$

norme: ≤40%

— après 4 heures $\dfrac{(a\,1 + a\,2 + b) \times 100}{X}$

norme: $\leq 70\%$

— après 8 heures $\dfrac{(a\,1 + a\,2 + b + c) \times 100}{X}$

norme: $\leq 85\%$

— après 12 heures $\dfrac{(a\,1 + a\,2 + b + c + d) \times 100}{X}$

norme: $\leq 100\%$

Pour illustrer la présente invention, on mentionnera ci-après un exemple de composition de microgranules répondant à une forme à délitement programmé dont le temps de dissolution total théorique in vitro est de 12 heures.

### Exemple de gélule dosée à 5 mg de substance active

Présentation
- microgranules à libération programmée
- gélules taille n° 2

dosage pour une gélule:
 5 mg dihydroergotamine méthanesulfonate

répartition théorique du principe actif pour une gélule
- fraction instantanée     15% (0,75 mg)
- fraction »Retard«     85% (4,25 mg) sur 12 h.

formule:

| | |
|---|---|
| — D.H.E. méthanesulfonate | 5,0 mg |
| — saccharose | 189,0 mg |
| — amidon de mais | 63,0 mg |
| — acide stéarique | 0,3 mg |
| — Polyvinyl pyrrolidone excipient | 2,7 mg |
| — lactose | 18,0 mg |
| — talc | 33,0 mg |
| — polymère méthacrylique L | 19,0 mg |
| poids théorique d'une gélule | 330,0 mg |

Mode opératoire

- Dans un mélangeur en acier inoxydable, on introduit des coeurs neutres de micro-granules constituées de saccharose (75%) et d'amidon de mais (25%);
- On projette sur ces micro-granules la solution alcoolique de polyvinyl pyrrolidone excipient;
- On incorpore à ces micro-granules la dihydroergotamine méthanesulfonate et les autres excipients;
- Enfin, on projette sur ces granules les polymères méthacryliques en solution dans l'alcool;
- On sèche, puis on élimine l'alcool, et
- Après les contrôles, on procède à la mise en gélules.

De nombreuses expérimentations ont été conduites avec diverses compositions dont on mentionnera uniquement les quelques exemples suivants qui permettront de mettre en lumière l'amélioration considérable de l'effet pharmacologique obtenu avec les compositions selon la présente invention.

### Exemple 1: (Courbe A)

Forme standard immédiate de méthanesulfonate de dihydroergotamine en gouttes.

## Exemple 2: (Courbe B)

Gélules de 5 mg de méthanesulfonate de dihydroergotamine comprenant 100% de micro-granules entièrement à libération instantanée.

## Exemple 3: (Courbe C)

Gélules de 5 mg de méthanesulfonate de dihydroergotamine comprenant:

50% de micro-granules retard sur 8 heures,
50% de micro-granules entièrement à libération instantanée.

## Exemple 4: (Courbe D)

Gélules de 5 mg de méthanesulfonate de dihydroergotamine comprenant:

85% de micro-granules retard sur 10 heures,
15% de micro-granules entièrement à libération instantanée.

## Exemple 5: (Courbe E)

Gélules de 5 mg de méthanesulfonate de dihydroergotamine comprenant:

85% de micro-granules retard sur 12 heures,
15% de micro-granules entièrement à libération instantanée.

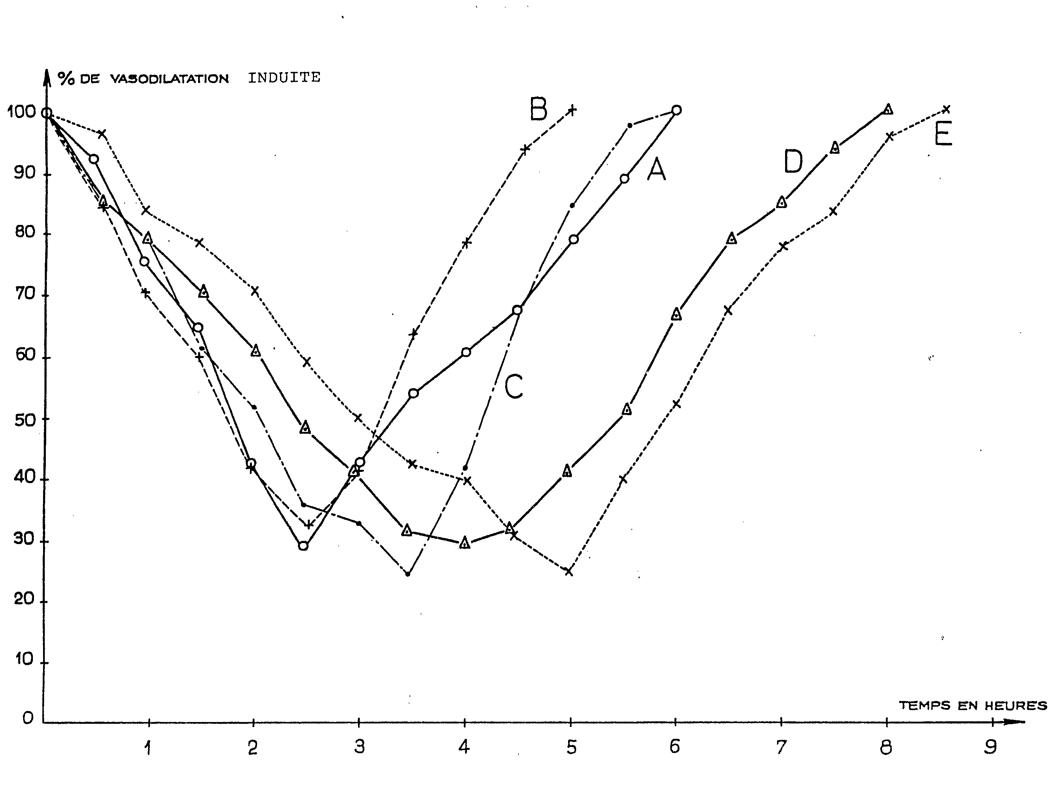

Les résultats expérimentaux obtenus avec les exemples précités, figurant dans le tableau ci-après, sont illustrés sur le dessin annexé sous la forme de courbes représentatives de la réduction, due à la drogue, de la vasodilatation induite par rapport au temps exprimé en heures. L'?evaluation de l'effet veino-constricteur de la drogue chez l'homme est basée sur la mesure de la compliance des veines du dos de la main, selon la méthode décrite par W. H. Aellig »Venoconstrictor Effect of Dihydroergotamine in Superficial Hand Veins«-Europ. Journ. of clin. Pharmacology. 7, 137 – 139 (1974).
La méthode est fondée sur l'évaluation des variations du diamètre veineux sous l'action de la drogue.
Les mesures sont faites à l'aide d'un système optique.
Lors de chaque mesure, la veine est soumise à une pression de valeur constante. Celle-ci est obtenue par la position constante de la main au-dessus du niveau central et la pose d'un brassard maintenu à 40 mg Hg.
Sur la figure annexée la courbe A représente les résultats obtenus avec une forme standard immédiate de méthanesulfonate (ci-après dénommé dihydroergotamine) en gouttes qui constitue la forme la plus largement utilisée à l'heure actuelle.
Au cours des expériences conduites dans le cadre de la présente invention, on a été amené à expérimenter la dihydroergotamine sous des formes solides de microgranulés dont on sait par ailleurs qu'ils présentent une plus grande stabilité dans le temps que les formes liquides correspondantes.
L'examen des courbes B et C de la figure annexée démontre clairement que les effets pharmacologiques angendrés restaient de même ordre que ceux obtenus par la forme standard en gouttes et ceci malgré l'introduction de 50% de dihydroergotamine sous forme retard (courbe C).
De Façon générale, on observera que dans le cas des exemples 1 à 3 correspondant respectivement aux courbes A, B et C, la durée de l'effet pharmacologique est limitée à 6 heures, ce qui nécessiterait en théorie au moins 4 prises de mèdicament par 24 heures, afin d'assurer un effet pharmacologique continu.

Tableau de resultats de reduction de la vasodilatation induite

(base 100 : vasodilatation maximale sous 40 mm Hg sans action de la drogue)

| Temps (h) | Exemple 1 | Exemple 2 | Exemple 3 | Exemple 4 | Exemple 5 |
|-----------|-----------|-----------|-----------|-----------|-----------|
| 0 | 100 | 100 | 100 | 100 | 100 |
| 0,5 | 93 | 85 | 85 | 85 | 96 |
| 1 | 75 | 70 | 79 | 79 | 84 |
| 1,5 | 64 | 61 | 61 | 70 | 78 |
| 2 | 43 | 42 | 52 | 61 | 71 |
| 2,5 | 29 | 33 | 36 | 48 | 59 |
| 3 | 43 | 42 | 33 | 42 | 50 |
| 3,5 | 54 | 64 | 24 | 33 | 43 |
| 4 | 61 | 79 | 42 | 30 | 40 |
| 4,5 | 68 | 94 | 67 | 33 | 31 |
| 5 | 79 | 100 | 85 | 42 | 25 |
| 5,5 | 89 | — | 97 | 52 | 40 |
| 6 | 100 | — | 100 | 67 | 53 |
| 6,5 | — | — | — | 79 | 68 |
| 7 | — | — | — | 85 | 78 |
| 7,5 | — | — | — | 94 | 84 |
| 8 | — | — | — | 100 | 96 |
| 8,5 | — | — | — | — | 100 |

Evaluation de l'effet veino-constricteur en fonction du temps selon W. H. Aellig (op. cit.)

En revanche, les compositions selon la présente invention ont permis d'accroitre considérablement la durée de l'activité pharmacologique du médicament, tout en conservant la même quantité de principe actif et sans diminuer pour autant le taux maximum de vasoconstriction.

Les courbes D et E illustrent les résultats obtenus avec des compositions selon la présente invention. On observe que la durée d'activité est portée à 8 heures et au-delà, c'est-à-dire ne nécessiterait en théorie plus que 3 prises du médicament par 24 heures.

On constate également que le taux maximum de vasoconstriction est identique à celui observé pour les compositions de la technique antérieure et que l'activité globale du médicament (mesurée sensiblement par la surface de la courbe située sous la droite correspondant à une vasodilatation 100%) a été augmentée de plus d'environ 60% par rapport à l'activité de la composition de l'exemple 1.

Au cours des diverses expériences effectuées, il a été démontré que la cinétique de l'effet pharmacologique était fonction de plusieurs facteurs et principalement:

— du pourcentage de principe actif mis immédiatement à la disposition de l'organisme, et
— de la durée du temps de libération du reste du principe actif sous forme retard par la forme galénique particulière.

Les courbes obtenues avec les compositions selon la présente invention respectent parfaitement ces deux facteurs essentiels.

Bien entendu, la présente invention ne se trouve pas limitée aux modes de réalisation particuliers

**0 036 350**

décrits, mais il est parfaitement possible, sans pour autant sortir du cadre de la présente invention, d'en imaginer un certain nombre de variantes.

**Revendications**

1. Composition pharmaceutique pour régulariser le tonus vasculaire, sous forme de doses unitaires de 3 à 10 mg d'un principe actif qui est un alcaloide de l'ergot de seigle ou un de ses sels et ayant un effet étalé dans le temps, caractérisée en ce que ledit principe actif se présente sous forme de micro-granulés, les uns contenant 10 à 20% en poids de la dose totale du principe actif étant à libération instantanée, les autres, contenant de 80 à 90% en poids du principe actif, étant protégés par plusieurs enrobages gastrosolubles et gastro-résistants à délitement programmé en fonction du pH et ayant un temps de dissolution totale théorique in vitro compris entre 10 et 14 heures.

2. Composition selon la revendication 1, caractérisée en ce que le principe actif est constitué par un alcaloide de l'ergot de seigle présentant un fragment acide lysergique dihydrogéné, ou l'un de ses sels.

3. Composition selon la revendication 2, caractérisée en ce que le principe actif est constitué par la dihydroergotamine ou l'un de ses sels.

4. Composition selon la revendication 3, caractérisée en ce que le principe actif est constitué par le méhtansulfonate de dihydroergotamine.

5. Composition selon l'une des revendications 1 à 4, caractérisée en ce que les doses unitaires se présentent sous la forme de comprimés ou de capsules dures.

6. Composition selon l'une des revendications 1 à 5, caractérisée en ce que le profil de libération du principe actif est le suivant:

| Pourcentage du principe actif libéré | temps de libération |
|---|---|
| 10 á 20% (de préférence de 10 à 16%) | après 10 minutes |
| ≤ 40% | après 1 heure |
| ≤ 70% | après 4 heures |
| ≤ 85% | après 8 heures |
| ≤100% | après 12 heures |

7. Composition selon l'une des revendications 1 à 6, caractérisée en ce qu'elle se présente sous la forme de doses unitaires contenant chacune environ 5 mg de méthanesulfonate de dihydroergotamine réparti à raison d'environ 85% en poids de principe actif présenté sous une forme protégée par plusieurs enrobages gastro-solubles et gastro-résistants à délitement programmé en fonction du pH et ayant un temps de dissolution total théorique in vitro de l'ordre de 12 heures, et d'environ 15% en poids de principe actif présenté sous une forme entièrement à libération instantanée.

**Patentansprüche**

1. Pharmazeutische Zusammensetzung zur Regulierung des Blutgefäß-Tonus in Form von Normaldosen zwischen 3 und 10 mg eines aktiven Bestandteiles, welcher ein Mutterkornalkaloid oder eines seiner Salze ist, und mit Retardwirkung, dadurch gekennzeichnet, daß der genannte aktive Bestandteil die Form von Mikrogranulat aufweist, wobei ein Teil enthaltend 10 bis 20 Gew.-% der Gesamtdosis des aktiven Bestandteiles sofort freigesetzt wird und der andere Teil enthaltend 80 bis 90 Gew.-% des aktiven Bestandteiles durch mehrere im Magen lösliche und im Magen resistente Hüllen geschützt ist, die sich in Abhängigkeit des pH-Wertes programmiert zersetzen, und eine theoretische Gesamtlösungszeit in vitro zwischen 10 und 14 Stunden hat.

2. Zusammensetzung nach Anspruch 1, dadurch gekennzeichnet, daß der aktive Bestandteil ein Mutterkornalkaloid ist, welches ein Fragment der Dihydrolysergsäure oder eines ihrer Salze darstellt.

3. Zusammensetzung nach Anspruch 2, dadurch gekennzeichnet, daß der aktive Bestandteile dihydroergotamin oder eines seiner Salze ist.

4. Zusammensetzung nach Anspruch 3, dadurch gekennzeichnet, daß der aktive Bestandteil

8

**0 036 350**

Dihydroergotaminmethansulfonat ist.

5. Zusammensetzung nach einem der Ansprüche 1 bis 4, dadurch gekennzeichnet, daß die Normaldosen die Form von Tabletten oder harten Kapseln aufweisen.

6. Zusammensetzung nach einem der Ansprüche 1 bis 5, dadurch gekennzeichnet, daß das Freisetzungsprofil des aktiven Bestandteiles wie folgt ist:

| Prozente des freigesetzten aktiven Bestandteiles | Freisetzungszeit |
|---|---|
| 10 bis 20% (vorzugsweise zwischen 10 und 16%) | nach 10 Minuten |
| ≤ 40% | nach 1 Stunde |
| ≤ 70% | nach 4 Stunden |
| ≤ 85% | nach 8 Stunden |
| ≤100% | nach 12 Stunden |

7. Zusammensetzung nach einem der Ansprüche 1 bis 6, dadurch gekennzeichnet, daß sie in Form von Normaldosen verfügbar ist, wobei jede ungefähr 5 mg Dihydroergotaminmethansulfonat enthält, wovon ein Teil mit ungefähr 85 Gew.-% des aktiven Bestandteiles in einer durch mehrere Hüllen geschützten gastro-löslichen und gastro-resistenten Form vorhanden ist, der sich in Abhängigkeit vom pH-Wert programmiert zersetzt und eine theoretische Gesamtlösungszeit in vitro im Bereich von 12 Stunden hat, und der andere Teil mit ungefähr 15 Gew.-% des aktiven Bestandteiles in einer Form mit sofortiger Freisetzung vorhanden ist.

## Claims

1. A pharmaceutical composition for regulating vascular tone, in the form of unit doses of from 3 to 10 mg of an active principle which is an alkaloid of ergot of rye or one of the salts thereof and having a prolonged effect, characterised in that said active principle is in the form of micro-granules, of which some, containing from 10 to 20% by weight of the total dose of the active principle, are capable of instantaneous release, whilst others, containing from 80 to 90% by weight of the active principle, are protected by several gastro-soluble and gastro-resistant coatings of which the disintegration is programmed as a function of the pH and having a theoretical total dissolution time in vitro of from 10 to 14 hours.

2. A composition according to claim 1, characterised in that the active principle consists of an alkaloid of ergot of rye having a dihydrolysergic acid fragment, or one of the salts thereof.

3. A composition according to claim 2, characterised in that the active principle is formed by dihydroergotamine or by one of the salts thereof.

4. A composition according to claim 3, characterised in that the active principle is formed by dihydroergotamine methanesulphonate.

5. A composition according to one of claims 1 to 4, characterised in that the unit doses are in the Form of tablets or hard capsules.

6. A composition according to one of claims 1 to 5, characterised in that the release profile of the active principle is as follows:

9

| Percentage of active principle released | Release time |
| --- | --- |
| 10 to 20% (preferably from 10 to 16%) | after 10 minutes |
| ≤ 40% | after 1 hour |
| ≤ 70% | after 4 hours |
| ≤ 85% | after 8 hours |
| ≤100% | after 12 hours |

7. A composition according to one of claims 1 to 6, characterised in that it is in the form of unit doses, each containing about 5 mg of dihydroergotamine methanesulphonate distributed in a proportion of about 85% by weight of active principle in a form protected by several gastro-soluble and gastro-resistant coatings of which the disintegration is programmed as a function of the pH and having a theoretical total dissolution time in vitro of about 12 hours, and about 15% by weight of active principle entirely in instantaneous release form.